# EUROPÄISCHE PATENTSCHRIFT

(11) **EP 3 134 030 B1**
(45) Veröffentlichungstag und Bekanntmachung des Hinweises auf die Patenterteilung: **25.06.2025**
(21) Anmeldenummer: 15727538.9
(22) Anmeldetag: 20.04.2015
(51) Int. Cl.: A61F 2/16

(54) **BEHÄLTERSYSTEM ZUM ERWÄRMEN EINER INTRAOKULARLINSE**
CONTAINER SYSTEM FOR HEATING AN INTRAOCULAR LENS
SYSTÈME DE RÉCIPIENT POUR CHAUFFER UNE LENTILLE INTRAOCULAIRE

(30) Priorität: 22.04.2014 DE 102014005719
(43) Veröffentlichungstag der Anmeldung: 01.03.2017
(73) Patentinhaber: Iolution GmbH, 22761 Hamburg (DE)
(72) Erfinder: MAROSCHECK, Christoph, 22761 Hamburg (DE); BINDER, Helmut, 14167 Berlin (DE)
(74) Vertreter: Friderichs, Gunther
(86) Internationale Anmeldenummer: PCT/DE2015/000191
(87) Internationale Veröffentlichungsnummer: WO 2015/161837

(56) Entgegenhaltungen:
- EP-A1- 0 766 496
- WO-A1-01/78566
- DE-A1- 2 739 788
- DE-A1- 3 434 836
- US-A1- 2007 250 068
- US-A1- 2011 264 102
- US-A1- 2011 264 103
- US-B1- 6 398 789

## Beschreibung

Die vorliegende Erfindung betrifft ein Behältersystem zum Erwärmen und Aufbewahren einer zu implantierenden Intraokularlinse.

### Hintergrund der Erfindung

Intraokularlinsen stellen Linsenimplantate oder künstliche Linsen zum Ersatz der natürlichen Linse eines menschlichen Auges dar. Sie werden insbesondere als Ersatz für die unter einer Augentrübung (Katarakt) erkrankten Linsen des Auges eingesetzt. Mittels einer Operation werden die erkrankten Linsen entfernt und die Intraokularlinsen eingesetzt. Diese werden zum Beispiel mittels eines sogenannten Injektors in das Auge eingesetzt bzw. eingeführt. Hierbei ist es von Bedeutung, dass der Operationsschnitt, durch welchen eine Intraokularlinse implantiert wird, möglichst klein ist (zum Beispiel etwa 2,5 mm). Dadurch kann ein möglichst schneller und komplikationsfreier Heilungsprozess gewährleistet sein und gegebenenfalls ist auch keine Naht erforderlich.

Um Intraokularlinsen, die im Allgemeinen einen Durchmesser von ca. 5 bis 7 mm besitzen, implantieren zu können, müssen diese faltbar sein, damit sie durch den kleinen Schnitt von etwa 2,5 mm hindurchpassen.

Ein Injektor zum Falten und Einbringen einer gefalteten Linse in das menschliche Auge ist beispielsweise in der internationalen Patentanmeldung WO 2012/155887 A1 genannt. Dort ist ein Injektorsystem zum Implantieren bzw.

Einbringen einer zeitweilig gefalteten Intraokularlinse beschrieben, mit welchem die gefaltete Linse durch eine Schnittöffnung im Auge mit der erforderlichen Größe von etwa 2,5 mm in die Linsenkapsel des Auges einsetzbar ist.

In einer Ausführungsform wird das mit einer Linse bestückte Magazin zum Laden des Injektorsystems erst kurz vor der Operation mit dem Injektor verbunden. Die Bestückung des Magazins mit der Linse kann somit vorab unter kontrollierten Bedingungen, zum Beispiel von einem Hersteller für Linsen, durchgeführt werden kann. Das bestückte Magazin kann dann in einem Aufbewahrungsbehälter, beispielsweise in einem Blister, der vorzugsweise mit einer sterilen Flüssigkeit befüllt ist, steril gelagert werden. Vorrichtungen zum Erwärmen einer Intraokularlinse sind auch aus den Dokumenten US 2011/264102 A1 und US 6,398,789 B1 bekannt.

### Allgemeine Beschreibung der Erfindung

Vor dem vorstehend geschilderten Hintergrund liegt der vorliegenden Erfindung die Aufgabe zugrunde, ein Einbringen der Linse in das Auge noch weiter zu verbessern.

Insbesondere soll es möglich sein, eine zu implantierende Linse noch kleiner zusammenzurollen.

Gelöst werden diese Aufgaben durch das Behältersystem mit den Merkmalen des Patentanspruchs 1.

Vorteilhafte Ausführungsformen sind Gegenstand der Unteransprüche, der Beschreibung und/oder der Zeichnungen. Es hat sich dabei herausgestellt, dass die Temperatur der Linse für das Falten der Linse, das Einbringen der Linse in das Auge und/oder das Entfalten der Linse eine gewichtige Rolle trägt. Eine erwärmte Linse ist deutlich geschmeidiger und flexibler und kann kleiner zusammengerollt werden, so dass eine Schnittöffnung in dem Auge noch weiter verkleinert werden kann. Zudem kann sich eine warme Linse nach dem Einführen in das Auge wieder schneller entfalten.

Allgemein sieht die Erfindung daher vor, den im Stand der Technik beschriebenen Aufbewahrungsbehälter, nachfolgend kurz den Behälter derart weiterzuentwickeln, dass er mit einer Heizeinrichtung bereitgestellt wird.

Gemäß der Erfindung ist ein Behältersystem zum Erwärmen und Aufbewahren einer Linse vorgesehen, das die folgende Bestandteile umfasst: Einen Behälter zum Lagern wenigstens einer in einem Magazin vorgeladenen Linse und eine in den Behälter integrierte Heizeinrichtung zum Erwärmen der in dem Magazin vorgeladenen Linse in dem Behälter.

Gemäß der Erfindung ist die Linse eine in das Auge zu implantierende Intraokularlinse.

Im Gegensatz zum Stand der Technik wird das Magazin nicht in ein Wärmebad gelegt. Sondern gemäß der Erfindung wird der Behälter mit der Heizeinrichtung bereitgestellt oder hergestellt. Die Heizeinrichtung ist in den Behälter integriert. Heizeinrichtung und Behälter sind oder bilden eine funktionale Einheit.

Gemäß der zweiten Variante der Offenbarung wird der Behälter nicht direkt mit der Heizeinrichtung bereitgestellt. Der Behälter wird mit der Anschlusseinrichtung für die Heizeinrichtung bereitgestellt. Die Anschlusseinrichtung ist in den Behälter integriert. Anschlusseinrichtung und Behälter sind oder bilden eine funktionale Einheit. Die Heizeinrichtung wird in diesem Fall separat bereitgestellt. Sie kann zum Beispiel als eine Art Ladestation ausgeführt sein, mit der der Behälter über seine Anschlusseinrichtung verbunden werden kann. Erfindungsgemäß ist die Linse nicht als solches in dem Behälter gelagert oder lagerbar, sondern befindet sich in einem Magazin und/oder ist vorgeladen auf dem Injektor. Das Magazin ist zusammen mit der in dem Magazin gelagerten Linse, gegebenenfalls samt Injektor in dem Behälter positioniert oder positionierbar. Insbesondere ist es dadurch in der ersten Ausführung möglich, das Magazin nach dem Öffnen des Behälters direkt mit einem Injektor zu verbinden. In der zweiten Ausführung ist es möglich, den vorgeladenen Injektor in dem Behälter stabil zu positionieren, um das Magazin und die Linse erwärmen zu können.

In einer ersten Ausgestaltung ist in dem Behälter in seinem geschlossenen Zustand bereits ein Magazin mit Linse positioniert. Insbesondere ist das Behältersystem dadurch gekennzeichnet, dass das Magazin zusammen mit der Linse in dem geschlossenen Behälter positioniert ist. Das Magazin kann nach dem Öffnen des Behälters mit dem Injektor verbunden werden. Das ist insbesondere für hydrophile Linse geeignet, da diese in einer Lösung gelagert werden.

Ebenfalls können hydrophobe Linsen derart positioniert und gelagert werden. Beide Linsen können jedoch auch außerhalb des Behälters positioniert und gelagert werden, um im Vorfeld der Operation indirekt über den vorgeladenen Injektor in den Behälter operationsbereit positioniert und erwärmt zu werden. In einer zweiten Ausgestaltung ist in dem Behälter zunächst keine Linse vorhanden. Dieser kann aber bereits mit einer Flüssigkeit befüllt sein. Erst zum Erwärmen wird die im Magazin vorgeladene Linse in den dann geöffneten Behälter eingebracht. Das Magazin wird zusammen mit der in dem Magazin gelagerten Linse in den Behälter eingebracht und ist dabei bereits am Injektor vormontiert. Das Behältersystem ist dadurch gekennzeichnet, dass das Magazin zusammen mit der Linse in dem geöffneten Behälter, vorzugsweise sicher, positionierbar ist. Das Magazin ist dabei bereits mit dem Injektor verbunden. Das ist insbesondere für hydrophobe Linse geeignet, da sie zusammen mit dem Magazin und/oder dem Injektor gelagert werden können, vorzugsweise ohne sich in einer Lösung zu befinden.

In einer weiteren Ausführungsform weist der Behälter einen Aufnahmebereich für das Magazin und/oder einen Abschnitt des Injektors auf. Erfindungsgemäß ist der Aufnahmebereich im Wesentlichen entsprechend dem Magazin und/oder dem vorderen Abschnitt des Injektors ausgebildet. Der Aufnahmebereich ist gemäß der Erfindung im Wesentlichen passgenau für das Magazin und/oder einen Abschnitt des Injektors ausgebildet. Eine im Wesentlichen passgenaue Form verringert oder vermeidet die Gefahr von Beschädigungen an dem Injektor, dem Magazin und/oder der Linse, insbesondere wenn der Injektor in den Behälter eingeführt wird.

Gemäß der Erfindung wird der Behälter bereitgestellt als ein Standbehälter. Dies offenbart einen weiteren Vorteil des Behälters. Er bietet dem mit dem Magazin verbundenen Injektor einen sicheren Stand, in dem der nun vorgeladenene Injektor auf dem OP-Tisch anwendungsbereit bis auf Abruf durch den Anwender vorgehalten werden kann. Insbesondere kann, wie bereits vorstehend ausgeführt, die Linse oder die Linse zusammen mit dem Magazin in dem Behälter in einer Flüssigkeit aufbewahrt sein. Ein Beispiel für eine solche Aufbewahrungsflüssigkeit ist eine sterile Kochsalzlösung. Das Flüssigkeitsvolumen liegt beispielsweise in einer Größenordnung von kleiner als 20 ml oder kleiner als 10 ml, vorzugsweise von 4 ml bis 5 ml.

Die Heizeinrichtung ist vorzugsweise ausgebildet, die Linse auf eine Zieltemperatur in einem Bereich von etwa 25°C oder 30°C bis 40°C, vorzugsweise von 37°C bis 40°C, zu erwärmen. Das soll insbesondere ausgehend von einer Starttemperatur in einem Bereich von etwa 4°C bis etwa 25°C möglich sein. In einer Ausgestaltung soll die Zieltemperatur nach etwa 30 Sekunden erreicht sein und/oder die Zieltemperatur über eine Dauer von etwa 1 min bis etwa 10 min im Wesentlichen gehalten werden.

Das Behältersystem kann als Einmalsystem oder auch als Mehrwegsystem bereitgestellt werden. Dies ist insbesondere davon abhängig, ob die Heizeinrichtung nach der Verwendung zum Beispiel wieder aufgeladen werden kann. Beide Varianten können die Linse bereits enthalten bzw. diese vor der Anwendung zugeführt bekommen. Dies ist insbesondere davon abhängig, ob eine hydrophobe Linse oder eine hydrophile Linse verwendet wird.

In einer Ausführungsform umfasst die Heizeinrichtung einen Latentwärmespeicher. Dieser kann beispielsweise basierend sein auf gesättigten Salzlösungen, wie z.B. Natriumazetat, und/oder auf Paraffin. Ein Latentwärmespeicher bietet insbesondere die Vorteile, dass er günstig ist, einfach integriert werden kann, ohne externe oder interne Spannungsversorgung betrieben werden kann, auch ohne wesentliche Schädigung sterilisiert, z.B. autoklaviert , werden kann und/oder sogar wiederverwendet werden kann. Insbesondere kann der Latentwärmespeicher bei der Sterilisierung, insbesondere durch Autoklavieren, ohne eine separate Wärmezufuhr aufgeladen werden.

Gemäß einer weiteren Ausführungsform der Heizeinrichtung umfasst diese eine elektrische Heizeinrichtung. Die dazu erforderliche Spannungsversorgung soll vorzugsweise durch eine Batterie oder einen Akkumulator bereitgestellt werden. Vorzugsweise soll die Spannungsversorgung nicht durch eine externe Spannungsversorgung bereitgestellt werden. Diese Art des Erwärmens bietet insbesondere den Vorteil, dass eine Zieltemperatur präzise und über eine längere Dauer gehalten werden kann.

Die Heizeinrichtung kann an einer Außenseite des Behälters angeordnet werden. Die Heizeinrichtung wird zum Beispiel als eine Art Ladestation bereitgestellt, in welche der Behälter eingeführt und erwärmt werden kann. In dieser Ausgestaltung ist die Heizeinrichtung mit dem Behälter über seine Anschlusseinrichtung verbindbar.

In einer bevorzugten Ausgestaltung ist die Heizeinrichtung an einer Außenseite des Behälters angeordnet. Zum Beispiel ist die Heizeinrichtung in einer Ausführungsform in den Behälter integriert. Dadurch kann ein kompaktes System bereitgestellt werden. Vorzugsweise weist die Heizeinrichtung einen Schalter zum Aktivieren auf. Die Heizeinrichtung kann dabei manuell oder automatisch aktivierbar sein. In einer Ausführungsform sind die Heizeinrichtung und der Behälter derart miteinander gekoppelt oder koppelbar, dass beim Öffnen des Behälters die Heizeinrichtung aktiviert oder eingeschaltet wird. In einer weiteren Ausgestaltung sind die Heizeinrichtung und der Deckel des Behälters derart miteinander gekoppelt oder koppelbar, dass beim Öffnen oder beim Abziehen des Deckels die Heizeinrichtung aktiviert wird.

Wie bereits vorstehend ausgeführt wurde, ist der Behälter oder das Behältersystem in einer Ausführungsform standfest, auch dann, wenn der Injektor mit dem Magazin verbunden ist. Insbesondere um die Standfestigkeit und/oder die Handhabung des Behältersystems zu verbessern, besitzt die Unterseite des Behälters einen gegenüber einem mittleren Bereich des Behälters größeren Querschnitt.

In einer ersten Ausgestaltung weist der Behälter eine, vorzugsweise im Wesentlichen ebene, Unterseite und/oder eine gegenüber der Unterseite geneigte Oberseite auf.

In einer zweiten Ausgestaltung besitzt der Behälter eine gegenüber der Unterseite geneigte, vorzugsweise im Wesentlichen ebene, erste Seitenwand und/oder eine gegenüber der Unterseite geneigte, vorzugsweise eine Stufe aufweisende, zweite Seitenwand, welche der ersten Seitenwand gegenüber liegt. Insbesondere durch die Stufe kann der Behälter durch den Anwender beim Abreißen des Deckels sicher gehalten werden. Vorzugsweise schließen die ebene Seitenwand und die Unterseite einen Winkel α1 von 90° < α1 < 120° ein.

In einer dritten Ausgestaltung ist eine Längsachse L des Aufnahmebereichs gegenüber der Unterseite des Behälters geneigt. Vorzugsweise schließen hierbei die Längsachse L des Aufnahmebereichs und die Unterseite einen Winkel α2 von 90° > α2 > 60° ein. Eine Linse alleine, ein Magazin mit der Linse und/oder auch ein gesamtes Injektorsystem können in dem erfindungsgemäßen Behältersystem gelagert sein. Die Abmessungen und/oder die Formen der Behältersysteme sind unter anderem abhängig vom Design einer zu implantierenden Intraokularlinse und/oder eines Magazins. Das Behältersystem ist z.B. geeignet für alle weichen, faltbaren Intraokularlinsen. Diese sind zum Beispiel aufgebaut aus Acryl, Silikon und/oder Hydrogel. Das Behältersystem kann eingesetzt werden für hydrophile Linsen, insbesondere mit einem Wassergehalt von 22 % bis 50 %, und/oder für hydrophobe Linsen, die in der Regel weniger flexibel sind.

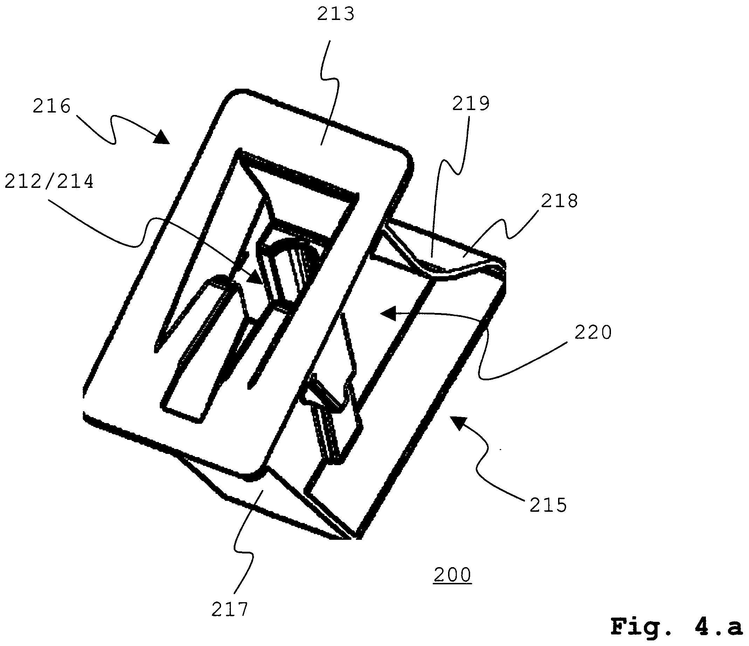

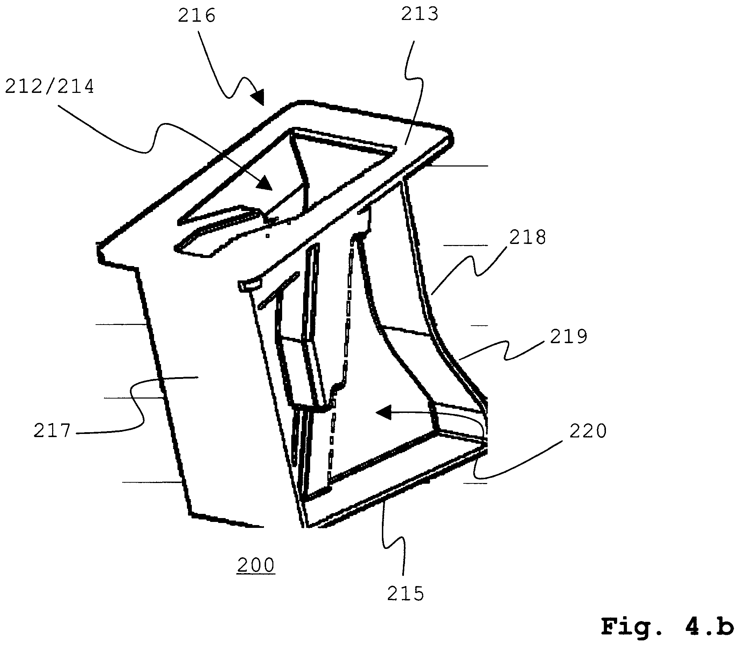

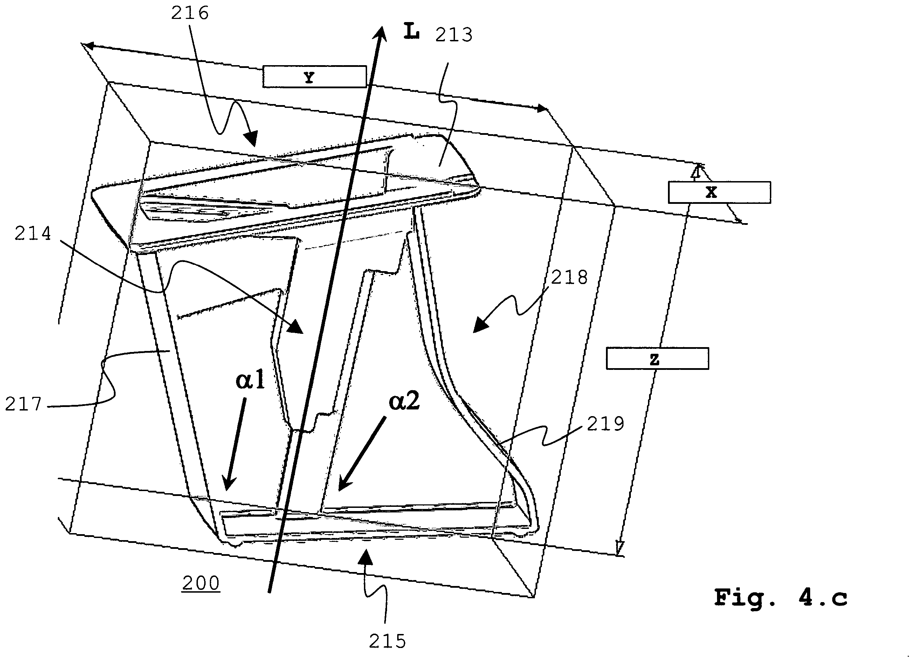

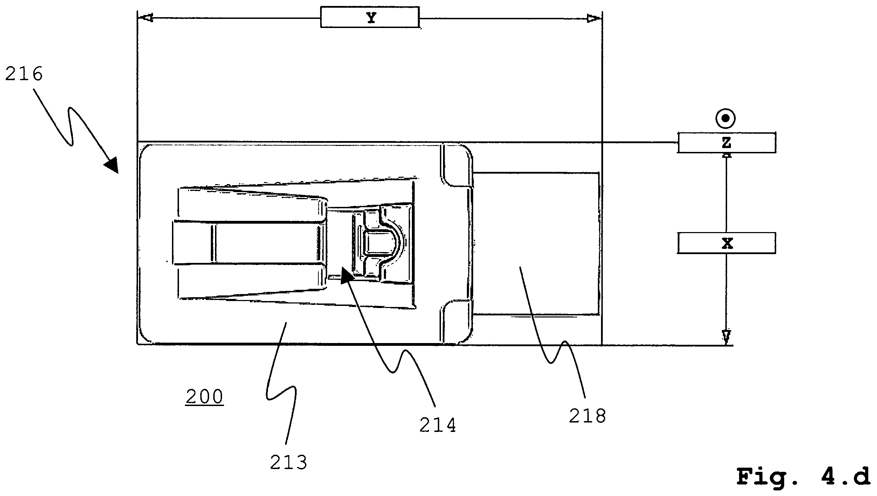

Zur Charakterisierung der Abmessungen werden die folgenden Parameter eingeführt (siehe dazu auch die Figuren 4.c und 4.d): Die Höhe Z, die Länge Y und die Breite X. Um z.B. ein kompaktes Behältersystem zu erhalten, können die folgenden Werte vorliegen: 20 mm < X < 50 mm, vorzugsweise 30 mm < X < 40 mm, und/oder 60 mm < Y < 100 mm, vorzugsweise 75 mm < Y < 85 mm, und/oder 50 mm < Z < 90 mm, vorzugsweise 60 mm < Z < 800 mm.

Der Behälter und/oder das Magazin sind in einer weiteren Ausführungsform der Erfindung im Wesentlichen transparent ausgeführt. Dadurch kann insbesondere das Einführen des Injektors in den Behälter und/oder in das Magazin visuell überprüft werden.

Die Heizeinrichtung und/oder die Anschlusseinrichtung für die Heizeinrichtung kann bzw. können in dem und/oder an dem Behälter integriert bereitgestellt werden. Es ist aber auch möglich, gewöhnliche Behälter mit einer Heizeinrichtung und/oder einer Anschlusseinrichtung für eine Heizeinrichtung nachzurüsten. Daher liegt im Bereich der Erfindung auch ein Bausatz zum Nachrüsten eines Behälters zum Aufbau eines Behältersystems gemäß wenigstens einer der vorstehend beschriebenen Ausführungsformen. Der Bausatz umfasst eine mit dem Behälter verbindbare Heizeinrichtung und/oder eine mit dem Behälter verbindbare Anschlusseinrichtung für eine Heizeinrichtung zum Erwärmen einer in einem Magazin vorgeladenen Linse in dem Behälter.

Die vorliegende Erfindung wird anhand der nachfolgenden Ausführungsbeispiele im Einzelnen erläutert. Hierzu wird auf die beigefügten Zeichnungen Bezug genommen. Die gleichen Bezugszeichen in den einzelnen Zeichnungen beziehen sich auf die gleichen Teile.

Fig. 1. zeigt ein Injektorsystem in seinem zusammengebauten Zustand mit geladenem Magazin.

Fig. 2 illustriert in einer perspektivischen Außenansicht das Laden des Injektorsystems mit dem Magazin, welches in einem schematisch dargestellten Behälter mit integrierter Heizeinrichtung positioniert ist. Fig. 3.a und 3.b illustrieren in einer perspektivischen Außenansicht das Anschließen des Behälters an eine nicht integrierte Heizeinrichtung. Fig. 4.a bis 4.d illustrieren in verschiedenen Ansichten den Behälter, in dem das Magazin aufbewahrt wird.

Fig. 5.a und 5.b illustrieren in einer perspektivischen Ansicht das vorzugsweise mit einem Magazin vorgeladene Injektionssystem in einem in das Behältersystem (hier durchsichtig dargestellt) eingeführten Zustand.

Fig. 6.a bis 6.e illustrieren in einer perspektivischen Ansicht das Einführen eines Injektors in das mit einem Magazin vorgeladene Behältersystem und dessen Entnahme.

Fig. 7 illustriert in einer perspektivischen Ansicht das Einführen eines mit einem Magazin vorgeladenen Injektors in das leere Behältersystem.

### Detaillierte Beschreibung der Erfindung:

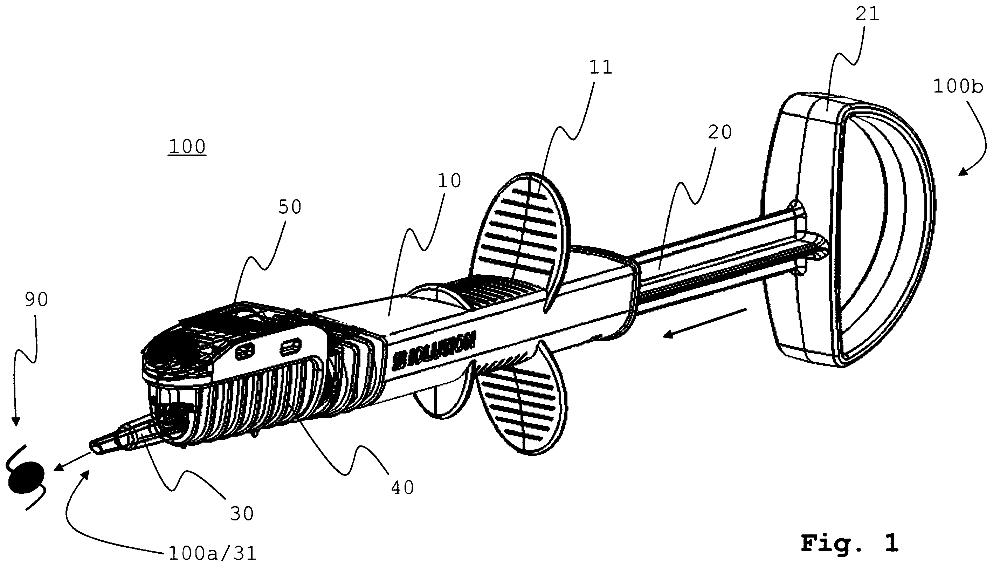

Figur 1 zeigt ein Injektorsystem 100 im zusammengebauten Zustand. Die Module zum Aufbau des Injektorssystems 100 umfassen ein Gehäuse 10, einen Schieber 20, eine Kanüle 30 und ein Magazin 40.

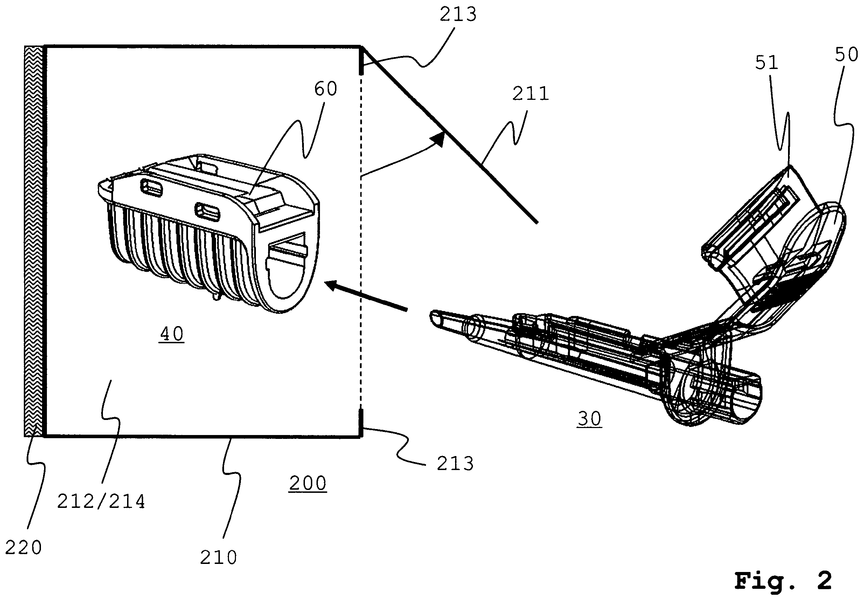

Auf der Kanüle 30 ist das Magazin 40 positioniert, hier vorzugsweise aufgesteckt. Beispielsweise sind die Kanüle 30 und das Magazin 40 einrastend miteinander verbunden. Das Magazin 40 wird vorzugsweise mit einer Linse 90 bestückt und die Linse 90 mit der Halteklappe 60 in dem Magazin 40 befestigt, bevor das Magazin 40 auf den Injektor 100 bzw. die Kanüle 30 aufgezogen wird (siehe dazu die Figur 2).

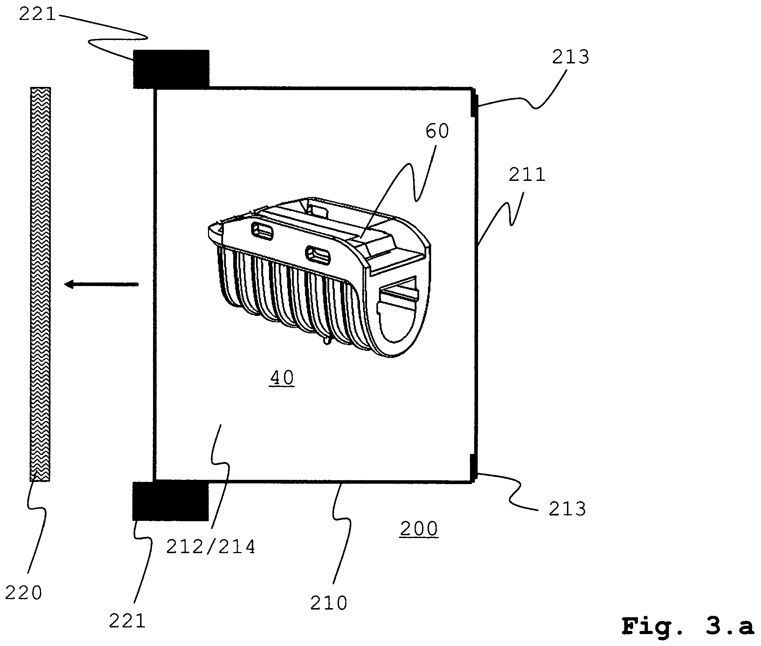

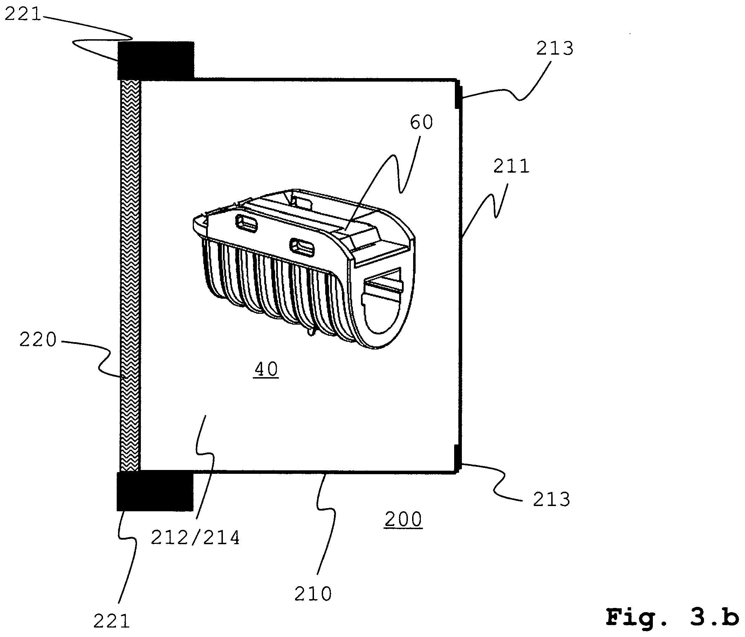

Vor der Inbetriebnahme des Injektors 100 wird zunächst der Aufbewahrungsbehälter 210 geöffnet. Dazu wird zum Beispiel der Deckel 211 von den Stirnflächen 213 abgezogen (siehe dazu Figur 2). Das Magazin 40 wird hier auf der Kanüle 30 aufgezogen, indem der Injektor 100 zumindest über seine Kanüle 30 in den Aufbewahrungsbehälter 210 eintaucht und das Magazin 40 auf die Kanüle 30 aufgesteckt wird. Dies bietet die Vorteile, dass die sterilen Bedingungen lange erhalten bleiben und der Transportkanal der Kanüle 30 mit der Aufbewahrungsflüssigkeit 212 benetzt ist, so dass die Linse 90 und der Schieber 20 besser in der Kanüle 30 gleiten können. Zudem ist in Figur 2 die erfindungsgemäße Heizeinrichtung 220 schematisch dargestellt. Beispielhaft dargestellt ist ein Behältersystem 200 gemäß einer ersten Ausführungsform aus einem Behälter 210 mit integrierter Heizeinrichtung 220. Die Heizeinrichtung 220 ist an der Außenseite des Behälters 210 positioniert. Die Heizeinrichtung 220 ist in den Behälter 210 integriert. Der Behälter 210 und die Heizeinrichtung 220 sind oder bilden eine funktionale Einheit. Zum Beispiel können das Material für einen Latentwärmespeicher in einem Hohlraum des Behälters 210, beispielsweise auf der linken und/ oder rechten Seite, eingefüllt sein und insbesondere ein Metallplättchen (nicht dargestellt) in der Außenwand des Behälters 210 eingebracht sein. Das Plättchen übernimmt die Funktion eines Schalters. Über ein Drücken des Plättchens wird das Erwärmen in Gang setzt. Das Aktivieren kann zum Beispiel auch über eine nicht in den Figuren dargestellte Kopplung bei der Öffnung des Deckels 211 erfolgen. Vorzugsweise sieht die Erfindung ein Behältersystem 200 zum Aufbewahren einer Linse 90 vor, umfassend einen Behälter 210 mit wenigstens einer in dem Behälter 210 gelagerten Linse 90 und eine Heizeinrichtung 220 zum Erwärmen der Linse 90 in dem Behälter 210. Das Erwärmen mittels der Heizeinrichtung 220 erfolgt über den Behälter 210. In Figur 2 ist die Heizeinrichtung 220 als eine mit dem Behälter 210, vorzugsweise dauerhaft, verbundene Einheit dargestellt. Die Heizeinrichtung 220 kann aber auch als eine Art Bausatz bereitgestellt werden, mit welchem ein gewöhnlicher Behälter 210 nachgerüstet werden kann. Dies ist den Figuren jedoch nicht dargestellt. Die Figuren 3.a und 3.b zeigen ein Behältersystem 200 gemäß einer zweiten Ausführungsform aus einem Behälter 210 mit integrierter Anschlusseinrichtung 221 für eine nicht integrierte Heizeinrichtung 220. Die Heizeinrichtung 220 wird zum Beispiel als eine Art Ladestation bereitgestellt, mit der der Behälter 210 über seine Anschlusseinrichtung 221 verbunden und dann erwärmt werden kann. Der Behälter 210 wird dazu zum Beispiel in Richtung der Heizeinrichtung 220 bewegt (Figur 3.a). Die Figur 3.b zeigt den finalen verbundenen Zustand.

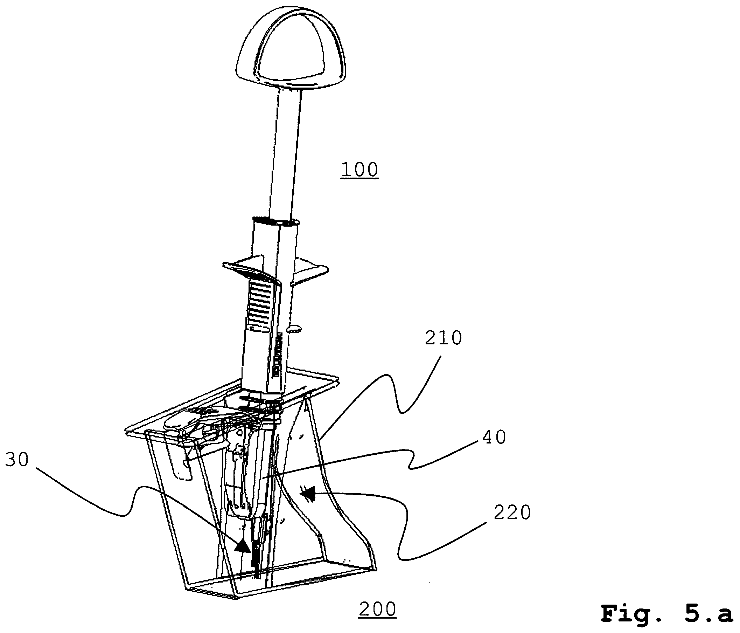

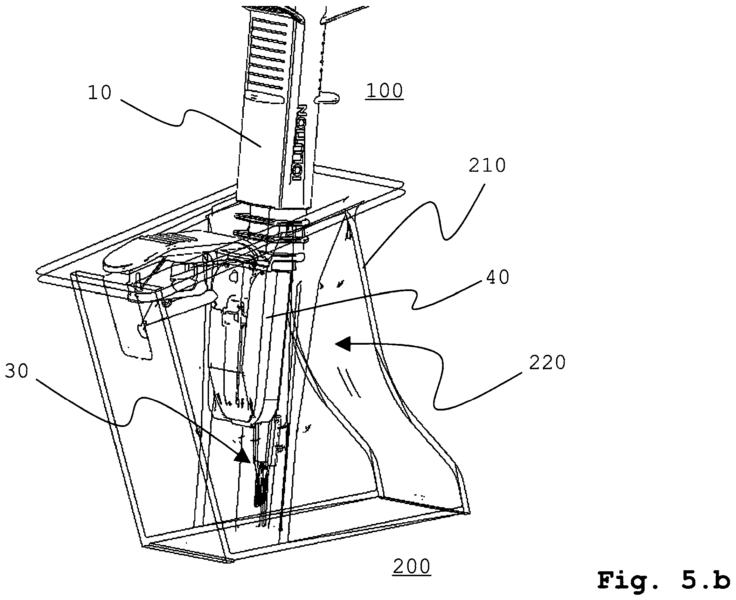

In den Figuren ist die Anschlusseinrichtung 221 als eine mit dem Behälter 210, vorzugsweise dauerhaft, verbundene Einheit dargestellt. Die Anschlusseinrichtung 221 kann aber auch, insbesondere in Verbindung mit der Heizeinrichtung 220, als eine Art Bausatz bereitgestellt werden, mit dem ein gewöhnlicher Behälter 210 nachgerüstet werden kann. Dies ist den Figuren jedoch nicht dargestellt. Die Figuren 4.a bis 4.d zeigen in unterschiedlichen Perspektiven einen leeren Behälter 210 für ein Magazin 40. Der Behälter 210 ist in verschiedenen Perspektiven dargestellt mit bereits abgezogenem Deckel 211. Der Behälter 210 ist als Standelement 210 ausgeführt. Das Standelement 210 dient dem vorgeladenen Injektionssystem 100 als Ablage oder zur Zwischenaufbewahrung (siehe dazu auch die Figuren 5.a und 5.b). Das Magazin 40 kann über ein Einführen des Injektors 100 in den Behälter 210 auf den Injektor 100 aufgezogen werden. In den Seitenwänden befinden sich Einbuchtungen, in denen eine hier nicht dargestellte Heizeinrichtung 220 positioniert werden kann. Das Material des Behälters 210 umfasst z.B. Polypropylen. Der Deckel 211 wird beispielsweise durch ein Metall, vorzugsweise Aluminium, bereitgestellt. Ein solches geschlossenes Behältersystem 200 mit Behälter 210 und einem Latentwärmespeicher als Heizeinrichtung 220 erfüllt auch die Anforderungen der Autoklavierbarkeit. Der Deckel kann auch weggelassen werden bzw. semipermeabel ausgeführt sein, so dass Anforderungen weiterer Sterilisationsmethoden, wie z.B. ETO oder Gammastrahlung, erfüllt werden.

Die Figuren 4.c (in einer perspektivische Seitenansicht) und 4.d (Aufsicht auf die Oberseite 216) zeigen die Maße eines erfindungsgemäßen Behältersystems 200. Dazu werden hier die Höhe Z, die Länge Y und die Breite X eingeführt. Insbesondere um ein kompaktes Behältersystem 200 zu haben, können die folgenden Werte vorliegen: 33 mm < X < 37 mm und/oder 77 mm < Y < 83 mm und/oder 67 mm < Z < 71 mm. Die Z-Achse verläuft in Figur 4.d senkrecht zur Blattebene.

Insbesondere um die Standfestigkeit und/oder die Handhabung zu verbessern, sind die Oberseite 216 sowie die Seitenwände 217 und 218 gegenüber der Unterseite 215 des Behälters 200 geneigt. Die Seitenwand 217 ist um einen Winkel α1 geneigt gegenüber der Unterseite 215. Auch ein oberer Abschnitt der Seitenwand 218 ist um den Winkel al geneigt gegenüber der Unterseite 215. Vorzugsweise beträgt 95° < α1 < 110°. Auch ist hier die Längsachse L des Aufnahmebereichs 214 um einen Winkel α2 geneigt gegenüber der Unterseite 215. Der Winkel a2 beträgt vorzugsweise 85° > α2 > 75°. Die Unterseite 215 des Behälters 210 hat gegenüber einem mittleren Bereich des Behälters 210 einen größeren Querschnitt. Insbesondere dazu ist in der Seitenwand 218 eine Stufe 219 vorgesehen. Durch die seitlich vorstehende Stufe 219 kann der Behälter 210 sicher von einem Anwender gehalten werden.

Die Figuren 5.a und 5.b illustrieren das Behältersystem 200 mit eingeführtem Magazin 60 und Injektor 100. Der Behälter 210 ist als ein Standelement ausgeführt. Er ist standfest. D.h. bei nicht eingeführtem und bei eingeführtem Injektor 100 trifft das Schwerpunktlot des Behältersystems die Auflagefläche des Behältersystems 200 und/oder die Auflagefläche des Behälters 210. Das Behältersystem kann mit dem eingeführten Injektor für die Anwendung abrufbereit und standsicher, z.B. auf dem Operationstisch, vorgehalten werden. Das Magazin 60 kann zusammen mit der Linse 90 bereits in dem geschlossenen Behälter 210 gewesen sein.

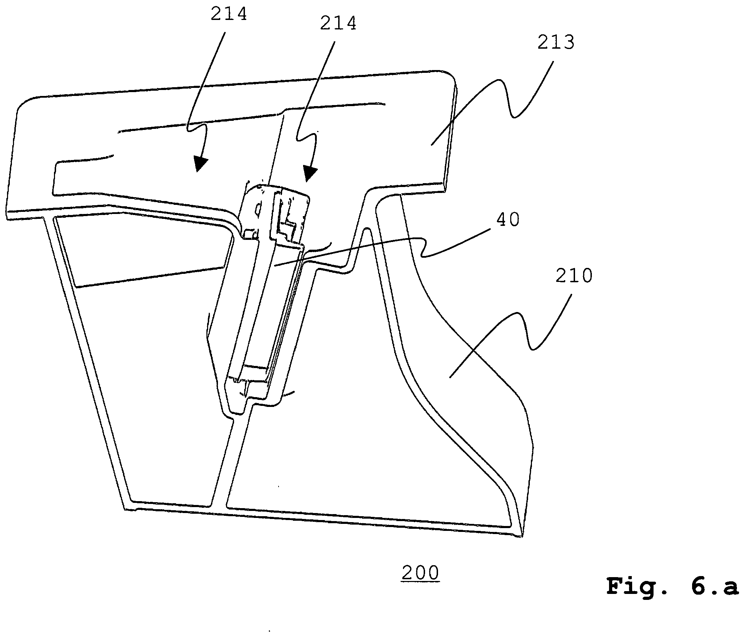

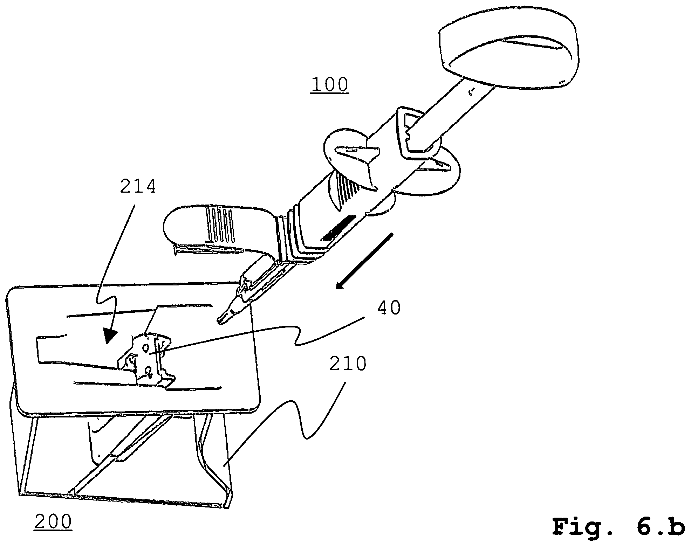

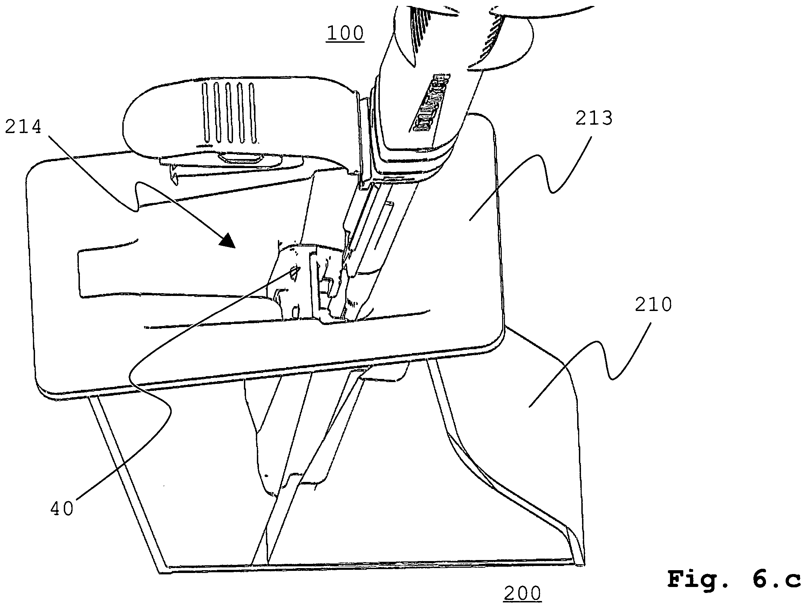

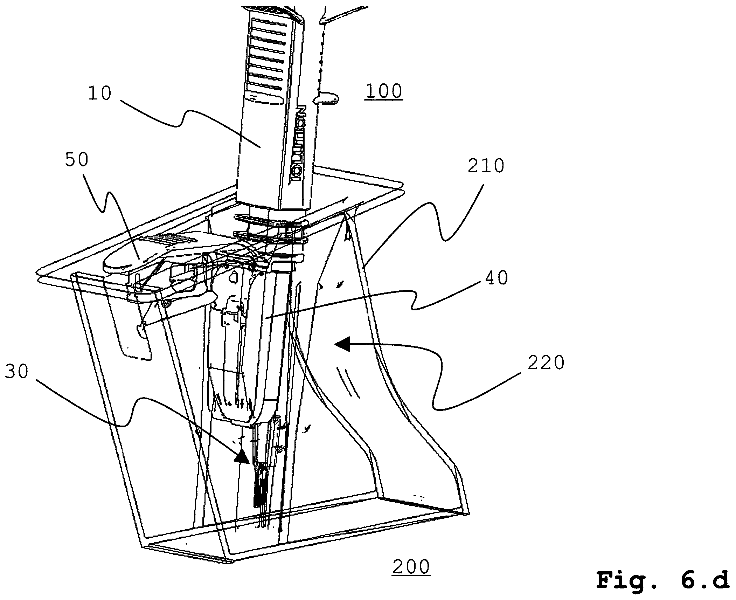

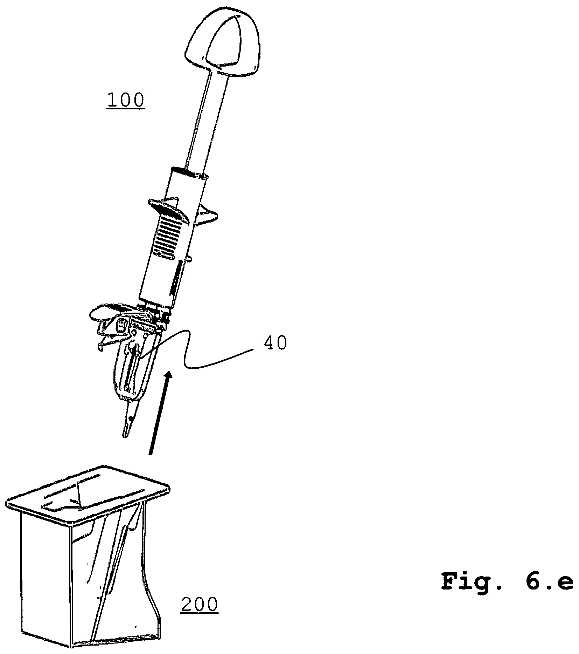

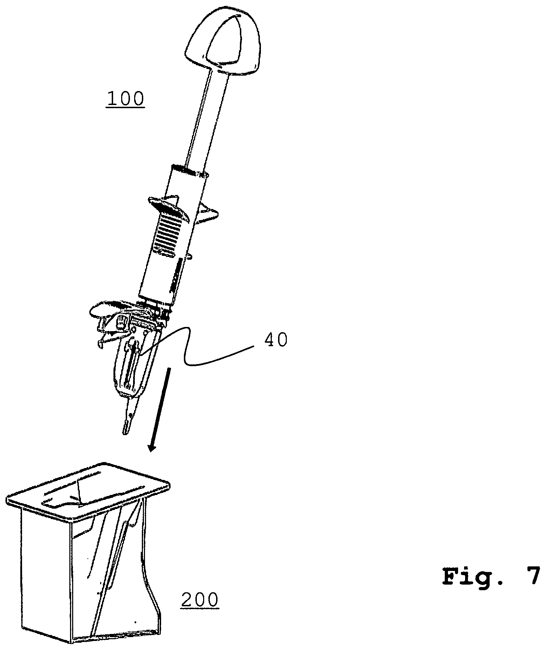

Nach dem Öffnen des Behälters 210 wurde dann der Injektor 100 in das Magazin eingeführt (siehe dazu die Figuren 6.a bis 6.e). Der geschlossene Behälter 210 kann aber auch zunächst ohne Linse 90 bereitgestellt worden sein. Das Magazin 60 wurde dann zusammen mit der Linse 90 und dem Injektor 100 in den geöffneten Behälter 210 zum Erwärmen eingeführt (siehe dazu Figur 7,). In den Figuren 5.a und 5.b wird ein weiterer Vorteil dargestellt. Neben der Erwärmung der Linse 90 zur gesteigerten Flexibilität derselben wird auch die Kanüle 30 auf die Zieltemperatur erwärmt. Die somit erhöhte Flexibilität der Kanüle 30 trägt ihrerseits zu einer Verbesserung des Operationsablaufs bei, so dass eine Verkleinerung des Schnitts bzw. das leichtere Einbringen von sonst weniger flexiblen Linsen 90 ermöglicht wird. Dies kann auch unterstützt werden durch den Einsatz von gleitverbessernden Verfahren. Letztere können in einem "Coating"- bzw. "Blooming"-Verfahren erreicht werden.

Die Figuren 6.a bis 7 illustrieren abschließend noch einmal verschiedene Konfigurationen, in denen das Behältersystem 200 bereitgestellt oder betrieben werden kann.

Zunächst zeigen die Figuren 6.a und 6.e das Einführen eines Injektors 100 in ein Behältersystem 200, welches mit einem Magazin 40 vorgeladen ist. Figuren 6.a zeigt den Behälter 210 mit bereits abgezogenem Deckel 211. In Figur 6.b ist zudem der Injektor 100 dargestellt, welcher in Pfeilrichtung in den Behälter 210 und das Magazin 40 eingesteckt wird. Figur 6.c zeigt eine vergrößerte Darstellung mit dem angenäherten Injektor 100 kurz vor dem Verbinden. Figur 6.d entspricht Figur 5.b und zeigt den Injektor 100 in dem mit dem Magazin 40 verbundenen Zustand. Zu erkennen ist, dass nicht nur das Magazin 40 in dem Aufnahmebereich 214 aufgenommen wird. Es wird auch ein Teil des Injektors in dem Aufnahmebereich 214 aufgenommen, hier beispielhaft die Kanüle 30 und/oder der Faltkörper 50. Figur 6.e illustriert die Entnahme des nun mit dem Magazin 40 geladenen Injektors 100. Diese Variante ist insbesondere für hydrphile, aber auch für hydrophobe Linsen 90 geeignet. Abschließend illustriert Figur 7 ein leeres Behältersystem 200. Der Behälter 210 ist hier nicht mit einem Magazin 40 vorgeladen. Dagegen ist hier nun der Injektor 100 mit einem Magazin 40 bereits geladen. Der hier vorgeladene Injektor 100 wird zum Erwärmen der in dem Magazin 40 gelagerten Linse 90 über seine Vorderseite in Pfeilrichtung in den Behälter 210 eingeführt. Der Behälter 210 kann zum Beispiel leer oder mit einer Flüssigkeit 212 gefüllt sein. Dabei kann der Behälter 210 bereits vorab mit der Flüssigkeit 212 vorgefüllt sein oder erst nach dem Öffnen mit der Flüssigkeit 212 befüllt worden sein. Diese Variante ist insbesondere für hydrophobe Linsen 90 geeignet.

### Bezugszeichenliste:

- 10: Injektorkörper oder Injektorgehäuse oder Gehäuse oder Handstück
- 11: Griff am Injektorkörper
- 20: Schieber oder Kolben oder Linsenschieber
- 21: Griff oder Schiebergriff
- 30: Kanüle oder Einführrohr in das Auge oder Ausführkörper für die Linse
- 31: Transportkanal oder Vorschubkanal
- 40: Magazin oder Behälter zum Lagern der Linse
- 50: Faltklappe oder Faltplattenträger oder Klappe
- 51: Faltkörper oder Faltplatte oder Faltrippe
- 60: Halterung oder Halteklappe oder Klappe
- 90: Linse oder Intraokularlinse
- 100: Injektor oder Injektorsystem oder Applikator
- 100a: Vorderseite des Injektors
- 100b: Rückseite des Injektors
- 200: Behältersystem
- 210: Behälter
- 211: Deckel des Behälters
- 212: Aufbewahrungsflüssigkeit
- 213: Anlagefläche für den Behälterdeckel
- 214: Aufnahmebereich für das Magazin im Behälter und/oder für einen vorderen Abschnitt des Injektors
- 215: Unterseite des Behälters
- 216: Oberseite des Behälters
- 217: erste, vorzugsweise ebene, Seitenwand des Behälters
- 218: zweite Seitenwand des Behälters
- 219: Stufe oder Knick der zweiten Seitenwand
- 220: Heizeinrichtung
- 221: Anschlusseinrichtung für Heizeinrichtung

## Patentansprüche

1. Behältersystem (200) zum Erwärmen und Aufbewahren einer in ein Auge zu implantierenden Intraokularlinse (90), umfassend einen als Standbehälter ausgebildeten Behälter (210),
ein Magazin (40) mit wenigstens einer in dem Magazin (40) vorgeladenen Intraokularlinse (90)
und/oder einen Injektor (100), auf dem sich vorgeladen die Intraokularlinse befindet,
wobei der Behälter (210) zum Lagern wenigstens der in dem Magazin (40) und/oder dem Injektor (100) vorgeladenen Intraokularline (90) ausgebildet ist und eine in den Behälter (210) integrierte Heizeinrichtung (220) zum Erwärmen der Intraokularline (90) in dem Behälter (210) aufweist, und wobei das Magazin (40) oder der Injektor (100) zusammen mit der in dem Magazin (40) und/oder dem Injektor (100) gelagerten Intraokularline (90) in dem Behälter (210) positioniert ist, **dadurch gekennzeichnet, dass** der Behälter (210) einen Aufnahmebereich (214) für das Magazin (40) und/oder einen Abschnitt des Injektors (100) aufweist, welcher entsprechend dem Magazin (49) und/oder einem vorderen Abschnitt des Injektors (100) im wesentlichen passgenau ausgebildet ist.

2. Behältersystem (200) nach dem vorstehenden Anspruch, **dadurch gekennzeichnet, dass** sich die Intraokularline (90) vorgeladen auf dem Injektor (100) befindet und samt dem Injektor (100) in dem Behälter (210) positioniert ist.

3. Behältersystem (200) nach einem der vorstehenden Ansprüche, **dadurch gekennzeichnet, dass** das Magazin (40) in dem Behälter (210) in einem geschlossenen Zustand des Behälters (210) positioniert ist.

4. Behältersystem (200) nach einem der vorstehenden Ansprüche, **dadurch gekennzeichnet, dass** der Behälter (210) mit einer Flüssigkeit (212) zum Aufbewahren und zum Erwärmen der Linse (90) vorgefüllt ist.

5. Behältersystem (200) nach einem der vorstehenden Ansprüche, **dadurch gekennzeichnet, dass** die Heizeinrichtung (220) ausgebildet ist, eine in dem Magazin (40) gelagerte Linse (90) auf eine Temperatur in einem Bereich von 25°C bis 40°C zu erwärmen.

6. Behältersystem (200) nach einem der vorstehenden Ansprüche, **dadurch gekennzeichnet, dass** die Heizeinrichtung (220) einen Latentwärmespeicher oder eine elektrische Heizeinrichtung umfasst.

7. Behältersystem (200) nach einem der vorstehenden Ansprüche, **dadurch gekennzeichnet, dass** die Heizeinrichtung (220) an einer Außenseite des Behälters (210) angeordnet ist.

8. Behältersystem (200) nach einem der vorstehenden Ansprüche, **dadurch gekennzeichnet, dass** die Heizeinrichtung (220) einen Schalter zum Aktivieren aufweist, wobei die Heizeinrichtung (220) manuell oder automatisch aktivierbar ist.

9. Behältersystem (200) nach einem der vorstehenden Ansprüche, **dadurch gekennzeichnet, dass** die Heizeinrichtung (220) und der Behälter (210) derart miteinander gekoppelt sind, dass bei einem Anschließen oder einem Öffnen des Behälters (210) die Heizeinrichtung (220) aktivierbar ist.

10. Behältersystem (200) nach einem der vorstehenden Ansprüche, **dadurch gekennzeichnet, dass** der Behälter (210) einen Deckel (211) aufweist, wobei die Heizeinrichtung (220) und der Deckel (211) des Behälters (210) derart miteinander gekoppelt sind oder koppelbar sind, dass beim Öffnen des Deckels (210) die Heizeinrichtung (220) aktivierbar ist.

11. Behältersystem (200) nach einem der vorstehenden Ansprüche, **dadurch gekennzeichnet, dass** eine Unterseite (215) des Behälters (210) einen gegenüber einem mittleren Bereich des Behälters (210) größeren Querschnitt besitzt.

12. Behältersystem (200) nach einem der vorstehenden Ansprüche, **dadurch gekennzeichnet, dass** der Behälter (210) eine Unterseite (215) und eine gegenüber der Unterseite (215) geneigte Oberseite (216) aufweist, und dass der Behälter (210) eine gegenüber der Unterseite (215) geneigte erste Seitenwand (217) und eine gegenüber der Unterseite (215) geneigte zweite Seitenwand (218) hat.

13. Behältersystem (200) nach einem der vorstehenden Ansprüche, **dadurch gekennzeichnet, dass** der Behälter (210) einen einem Abschnitt des Injektors (100) entsprechenden Aufnahmebereich (214) für das Magazin (40) aufweist.

14. Behältersystem (200) nach einem der vorstehenden Ansprüche, **dadurch gekennzeichnet, dass** eine Längsachse (L) des Aufnahmebereichs (214) gegenüber der Unterseite (215) des Behälters (210) geneigt ist.

## Claims

1. A container system (200) for heating and storing an intraocular lens (90) to be implanted in an eye, comprising
a container (210) in the form of a standing container;
a cartridge (40) with at least one intraocular lens (90) preloaded in the cartridge (40) and/or an injector (100) on which the intraocular lens is preloaded;
wherein the container (210) is designed for storing at least the intraocular lens (90) preloaded in the cartridge (40) and/or in the injector (100) and comprises a heating means (220) integrated in the container (210) for heating the intraocular lens (90) inside the container (210), and wherein the cartridge (40) or the injector (100) is located in the container (210) together with the intraocular lens (90) accommodated in the cartridge (40) and/or in the injector (100);
**characterized in that** the container (210) has an accommodation area (214) for the cartridge (40) and/or for a portion of the injector (100), which is designed to fit substantially precisely to the cartridge (49) and/or to a front portion of the injector (100).

2. The container system (200) according to the preceding claim, **characterized in that** the intraocular lens (90) is preloaded on the injector (100) and is accommodated in the container (210) along with the injector (100).

3. The container system (200) according to any one of the preceding claims, **characterized in that** the cartridge (40) is accommodated in the container (210) in a closed state of the container (210).

4. The container system (200) according to any one of the preceding claims, **characterized in that** the container (210) is prefilled with a liquid (212) for storing and heating the lens (90).

5. The container system (200) according to any one of the preceding claims, **characterized in that** the heating means (220) is adapted for heating a lens (90) accommodated in the cartridge (40) to a temperature in a range from 25 °C to 40 °C.

6. The container system (200) according to any one of the preceding claims, **characterized in that** the heating means (220) comprises a latent heat storage or an electrical heating means.

7. The container system (200) according to any one of the preceding claims, **characterized in that** the heating means (220) is arranged at an outer surface of the container (210).

8. The container system (200) according to any one of the preceding claims, **characterized in that** the heating means (220) has a activation switch, wherein the heating means (220) can be activated manually or automatically.

9. The container system (200) according to any one of the preceding claims, **characterized in that** the heating means (220) and the container (210) are coupled to one another in such a way that the heating means (220) can be activated when the container (210) is connected or opened.

10. The container system (200) according to any one of the preceding claims, **characterized in that** the container (210) has a lid (211), wherein the heating means (220) and the lid (211) of the container (210) are or can be coupled to one another in such a way that the heating means (220) can be activated when the lid (210) is opened.

11. The container system (200) according to any one of the preceding claims, **characterized in that** a bottom side (215) of the container (210) is larger in cross section than a central portion of the container (210).

12. The container system (200) according to any one of the preceding claims, **characterized in that** the container (210) has a bottom side (215) and a top side (216) inclined relative to the bottom side (215), and **in that** the container (210) has a first side wall (217) inclined relative to the bottom side (215) and a second side wall (218) inclined relative to the bottom side (215).

13. The container system (200) according to any one of the preceding claims, **characterized in that** the container (210) has an accommodation area (214) for the cartridge (40), which corresponds to a portion of the injector (100).

14. The container system (200) according to any one of the preceding claims, **characterized in that** a longitudinal axis (L) of the accommodation area (214) is inclined relative to the bottom side (215) of the container (210).

## Revendications

1. Système de récipient (200) pour chauffer et stocker une lentille intraoculaire (90) à implanter dans un œil, comprenant
un récipient (210) conçu comme un récipient debout,
une cartouche (40) avec au moins une lentille intraoculaire (90) préchargée dans la cartouche (40)
et/ou un injecteur (100) sur lequel la lentille intraoculaire est préchargée,
dans lequel le récipient (210) est conçu pour stocker au moins la lentille intraoculaire (90) préchargée dans la cartouche (40) et/ou dans l'injecteur (100) et comprend un dispositif de chauffage (220) intégré dans le récipient (210) pour chauffer la lentille intraoculaire (90) dans le récipient (210), et dans lequel la cartouche (40) ou l'injecteur (100) est disposé dans le récipient (210) avec la lentille intraoculaire (90) stockée dans la cartouche (40) et/ou dans l'injecteur (100),
**caractérisé en ce que** le récipient (210) présente une zone de réception (214) pour la cartouche (40) et/ou pour une section de l'injecteur (100), qui est conçue pour s'adapter sensiblement précisément à la cartouche (49) et/ou à une section avant de l'injecteur (100).

2. Système de récipient (200) selon la revendication précédente, **caractérisé en ce que** la lentille intraoculaire (90) est préchargée sur l'injecteur (100) et est logée dans le récipient (210) avec l'injecteur (100).

3. Système de récipient (200) selon l'une quelconque des revendications précédentes, **caractérisé en ce que** la cartouche (40) est logée dans le récipient (210) dans un état fermé du récipient (210).

4. Système de récipient (200) selon l'une quelconque des revendications précédentes, **caractérisé en ce que** le récipient (210) est prérempli d'un liquide (212) pour stocker et chauffer la lentille (90).

5. Système de récipient (200) selon l'une quelconque des revendications précédentes, **caractérisé en ce que** le dispositif de chauffage (220) est conçu pour chauffer une lentille (90) stockée dans la cartouche (40) à une température comprise dans une plage de 25 °C à 40 °C.

6. Système de récipient (200) selon l'une quelconque des revendications précédentes, **caractérisé en ce que** le dispositif de chauffage (220) comprend un dispositif de stockage de chaleur latente ou un dispositif de chauffage électrique.

7. Système de récipient (200) selon l'une quelconque des revendications précédentes, **caractérisé en ce que** le dispositif de chauffage (220) est disposé sur un côté extérieur du récipient (210).

8. Système de récipient (200) selon l'une quelconque des revendications précédentes, **caractérisé en ce que** le dispositif de chauffage (220) comporte un interrupteur pour l'activation, le dispositif de chauffage (220) pouvant être activé manuellement ou automatiquement.

9. Système de récipient (200) selon l'une quelconque des revendications précédentes, **caractérisé en ce que** le dispositif de chauffage (220) et le récipient (210) sont couplés l'un à l'autre de telle manière que le dispositif de chauffage (220) peut être activé lorsque le récipient (210) est connecté ou ouvert.

10. Système de récipient (200) selon l'une quelconque des revendications précédentes, **caractérisé en ce que** le récipient (210) comporte un couvercle (211), le dispositif de chauffage (220) et le couvercle (211) du récipient (210) étant ou pouvant être couplés l'un à l'autre de telle manière que le dispositif de chauffage (220) puisse être activé lorsque le couvercle (210) est ouvert.

11. Système de récipient (200) selon l'une quelconque des revendications précédentes, **caractérisé en ce qu'**un côté inférieure (215) du récipient (210) présente une section transversale plus grande qu'une zone centrale du récipient (210).

12. Système de récipient (200) selon l'une quelconque des revendications précédentes, **caractérisé en ce que** le récipient (210) présente un côté inférieur (215) et un côté supérieur (216) inclinés par rapport au côté inférieur (215), et **en ce que** le récipient (210) présente une première paroi latérale (217) inclinée par rapport au côté inférieur (215) et une deuxième paroi latérale (218) inclinée par rapport au côté inférieur (215).

13. Système de récipient (200) selon l'une quelconque des revendications précédentes, **caractérisé en ce que** le récipient (210) présente une zone de réception (214) pour la cartouche (40) correspondant à une section de l'injecteur (100).

14. Système de récipient (200) selon l'une quelconque des revendications précédentes, **caractérisé en ce qu'**un axe longitudinal (L) de la zone de réception (214) est incliné par rapport au côté inférieure (215) du récipient (210).
